(19) Europäisches Patentamt
European Patent Office
Office européen des brevets

(11) Numéro de publication : **0 349 418 B1**

# (12) FASCICULE DE BREVET EUROPEEN

(45) Date de publication du fascicule du brevet : **09.09.92 Bulletin 92/37**

(51) Int. Cl.$^5$ : **C07D 261/18,** A61K 7/42, A61K 31/42

(21) Numéro de dépôt : **89401828.2**

(22) Date de dépôt : **27.06.89**

(54) **Dérivés d'hydroxy-4 isoxazoles, leur procédé de préparation et compositions cosmétiques et pharmaceutiques les contenant.**

(30) Priorité : **27.06.88 FR 8808612**

(43) Date de publication de la demande : **03.01.90 Bulletin 90/01**

(45) Mention de la délivrance du brevet : **09.09.92 Bulletin 92/37**

(84) Etats contractants désignés : **DE FR GB NL**

(56) Documents cités :
**US-A- 2 908 688**
**CHEMICAL ABSTRACTS, vol. 98, no. 11, 14 mars 1983, page 584, résumé no. 89659r, Columbus, Ohio, US**

(73) Titulaire : **L'OREAL**
**14, Rue Royale**
**F-75008 Paris (FR)**

(72) Inventeur : **Zysman, Alexandre**
**6, rue George Eastman**
**F-75013 Paris (FR)**
Inventeur : **Sebag, Henri**
**26, rue Erlanger**
**F-75016 Paris (FR)**

(74) Mandataire : **Stalla-Bourdillon, Bernard et al**
**CABINET NONY & CIE 29, rue Cambacérès**
**F-75008 Paris (FR)**

EP 0 349 418 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés d'hydroxy-4 isoxazoles en particulier des dérivés 3,5-disubstitués, leur procédé de préparation et leur utilisation en cosmétique et en thérapeutique.

Les nouveaux dérivés d'hydroxy-4 isoxazoles tels qu'ils seront définis ci-après sont particulièrement appropriés pour la réalisation de compositions cosmétiques destinées notamment à protéger la peau contre les effets néfastes du soleil.

Ces dérivés présentent en effet une absorption en U.V couvrant le domaine spectral compris entre 240 et 300nm de telle sorte qu'ils constituent de bons filtres solaires à l'égard des radiations ultraviolettes du type U.V-B qui sont à l'origine des inflammations ou erythèmes de la peau lorsque le corps humain est soumis au rayonnement solaire sans protection particulière.

Les premières études des propriétés thérapeutiques ont montré que ces composés étaient particulièrement prometteurs dans les applications thérapeutiques connues des dérivés d'isoxazole.

Par ailleurs, ces dérivés d'hydroxy-4 isoxazoles, compte-tenu de leurs fonctions réactives, sont susceptibles de constituer des intermédiaires très utiles pour la préparation de nouveaux composés actifs à action thérapeutique potentielle.

Les nouveaux dérivés d'hydroxy-4 isoxazoles selon l'invention peuvent être représentés par la formule générale suivante:

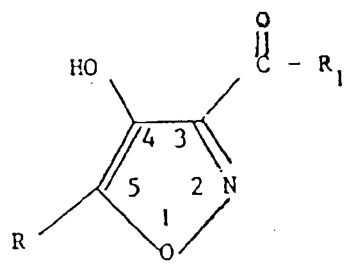

(I)

dans laquelle:

R représente un radical alkyle ou hydroxyalkyle, linéaire ou ramifié, ayant de 1 à 23 atomes de carbone, un radical alkényle, linéaire ou ramifié, ayant de 2 à 23 atomes de carbone, un radical phényle ou un radical benzyle, et

$R_1$ représente $OR_2$ , $-NHR_3$ ou $-NH-NHR_4$ ,

$R_2$ représentant un atome d'hydrogène ou un radical alkyle ayant de 1 à 24 atomes de carbone,

$R_3$ représentant un atome d'hydrogène, un radical alkyle ayant de 1 à 20 atomes de carbone ou un radical

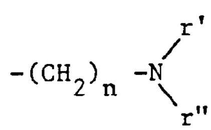

n étant 2 ou 3 et r' et r", identiques ou différents, représentant un atome d'hydrogène, un radical alkyle inférieur ou pris ensemble forment une morpholine, pipéridine ou pipérazine,

$R_4$ représentant un atome d'hydrogène ou un radical benzyle,

et les sels des composés de formule (I).

Lorsque le radical R représente un radical alkyle ayant de 1 à 23 atomes de carbone, celui-ci est de préférence un radical éthyle, propyle, hexyle, éthyl-2 hexyle, octyle, nonyle, dodécyle, tétradécyle ou octadécyle.

Selon une forme de réalisation préférée de l'invention le radical R est un radical alkyle ayant de 12 à 18 atomes de carbone, notamment le radical dodécyle, tétradécyle ou octodécyle.

Lorsque les composés selon l'invention se présentent sous forme de sels il s'agit plus particulièrement d'ammonium quaternaires obtenus par quaternisation des composés de formule (I) dans laquelle $R_1$ représente le radical $-NHR_3$ , $R_3$ représentant le radical

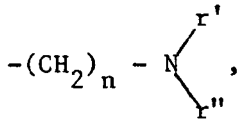

n, r' et r'' étant tels que définis ci-dessus, ces derniers ne pouvant toutefois représenter un atome d'hydrogène.

Parmi les agents de quaternisation, on peut notamment citer les halogénures de méthyle, le sulfate de diméthyle et le tosylate ou mésylate de méthyle.

Parmi les composés particulièrement préférés selon l'invention et correspondant à la formule (I) ci-dessus on peut notamment mentionner les suivants:

- le méthoxycarbonyl-3 hydroxy-4 tétradécyl-5 isoxazole,
- l'hydrazinocarbonyl-3 hydroxy-4 tétradécyl-5 isoxazole,
- le (diméthylamino-2 éthylamido)-3 hydroxy-4 tétradécyl-5 isoxazole (et son chlorhydrate),
- le méthylsulfate de (triméthylammonio-2 éthylamido)-3 hydroxy-4 tétradécyl-5 isoxazole,
- le n-butylamido-3 hydroxy-4 tétradécyl-5 isoxazole,
- l'éthoxycarbonyl-3 hydroxy-4 tétradécyl-5 isoxazole, et
- l'hexadécyloxycarbonyl-3 hydroxy-4 tétradécyl-5 isoxazole.

La présente invention a également pour objet le procédé de préparation des dérivés d'hydroxy-4 isoxazoles de formule (I) telle que définie ci-dessus.

Ce procédé est particulièrement avantageux dans la mesure où il permet d'accéder aux composés selon l'invention avec de bons rendements en une seule étape.

Ce procédé qui consiste à faire réagir sur un β-cétoester (1) du nitrite de butyle conduit, par une cyclisation spontanée résultant d'une déshydrogénation, aux alkoxycarbonyl-3 hydroxy-4 isoxazoles (2) selon le schéma réactionnel suivant:

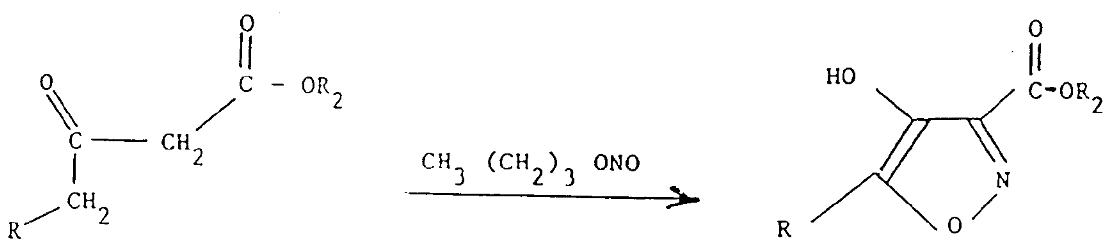


(1) (2)

$R_2$ = Alkyle $C_1$-$C_3$

La réaction est de préférence réalisée en solution dans un solvant tel que l'éther diéthylique ou l'éther diisopropylique, avec au moins 3 moles de nitrite de butyle par mole de β-cétoester (1) en présence de gaz chlorhydrique anhydre.

Plus précisément, le β-cétoester (1) et le nitrite de butyle sont mélangés dans le solvant à une température généralement inférieure à 20°C et de préférence comprise entre -10 et 10°C. A cette température on verse, sur le mélange réactionnel, une solution d'éther anhydre saturée de gaz chlorhydrique. Dès la fin de l'addition on cesse le refroidissement puis on abandonne le milieu à température ambiante pendant plusieurs heures. L'alkoxy carbonyl-3 hydroxy-4 isoxazole (2) est alors isolé selon les méthodes classiques de purification c'est-à-dire par distillation, cristallisation ou encore par chromatographie préparative sous pression.

On a noté que ce procédé conduisait à la formation comme produit secondaire d'un isomère des composés de formule (I), celui-ci correspondant à la formule suivante:

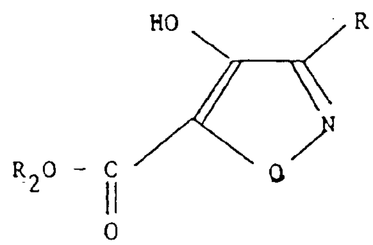

Cet isomère est généralement formé en une proportion n'excédant pas 20% en poids du produit réactionnel obtenu par le procédé selon l'invention.

Cet isomère du fait de la position de ces substituants n'absorbe pas le rayonnement U.V dans le domaine des longueurs d'onde présentant un intérêt en cosmétique.

Les composés selon l'invention de formule (I) dans laquelle $R_1$ représente $-NHR_3$ et $-NH-NH-R_4$ sont obtenus à partir des alkoxycarbonyl-3 hydroxy-4 isoxazoles selon les méthodes conventionnelles.

La présente invention a également pour objet des compositions cosmétiques pour la protection contre les radiations ultraviolettes contenant comme ingrédient actif, dans un véhicule cosmétique acceptable, au moins un dérivé d'hydroxy-4 isoxazole de formule (I) telle que définie ci-dessus.

Ces compositions peuvent se présenter sous la forme de solutions aqueuses ou hydroalcooliques, de solutions huileuses ou d'émulsions ou encore sous forme de sticks. Elles peuvent en outre être incorporées en association avec un agent propulseur et constituer des compositions sous forme d'aérosols.

Les compositions cosmétiques selon l'invention peuvent contenir en outre divers adjuvants habituellement présents dans les compositions cosmétiques anti-solaires à savoir des agents hydratants, émollients ou épaississants, des tensio-actifs, des agents de conservation, des parfums ou encore des pigments.

La concentration en dérivés d'hydroxy-4 isoxazole des compositions selon l'invention est généralement comprise entre 0,1 et 80% en poids par rapport au poids total de la composition.

On va maintenant donner à titre d'illustration plusieurs exemples de préparation des dérivés d'hydroxy-4 isoxazole de formule (I) ainsi que des exemples de compositions anti-solaires.

EXEMPLE 1 : Méthoxycarbonyl-3 hydroxy-4 tétradécyl-5 isoxazole.

1ère étape: Préparation de l'oxo-3 octadécanoate de méthyle.

En opérant constamment sous azote, on dissout 576g (4moles) d'acide de Meldrum (diméthyl-2,2 dioxane-1,3 dione-4,6) dans 2400cm$^3$ de dichlorométhane. A la solution ramenée à 0°C, on ajoute, goutte à goutte, 640cm$^3$ de pyridine.

Toujours à la même température on ajoute, en 1 heure, 1208g (4,4moles) de chlorure de palmitoyle puis on maintient le milieu sous agitation encore 1 heure à 0°C. On ajoute alors, toujours à 0°C, 6000cm$^3$ de méthanol et on maintenant encore le milieu à cette température pendant 1 heure puis on abandonne une nuit au repos.

Le lendemain on porte le milieu 5 heures à reflux puis l'on refroidit, essore, et sèche. On isole 1060g d'un solide blanc de point de fusion : 55°C.

2ème étape : Préparation du méthoxycarbonyl-3 hydroxy-4 tétradécyl-5 isoxazole.

Dans 80cm$^3$ d'éther anhydre on solubilise 15,6g (0,05mole) d'oxo-3 octadécanoate de méthyle (préparé ci-dessus) ainsi que 20,6g (0,2mole) de nitrite de butyle. Après avoir amené le milieu réactionnel à 0°C on ajoute en 1h30 au milieu réactionnel 200cm$^3$ d'une solution d'éther anhydre saturé de gaz chlorhydrique. On laisse revenir le milieu réactionnel à la température ambiante en maintenant l'agitation puis on l'abandonne au repos une nuit à cette même température. Le lendemain on isole par essorage 8g de méthoxycarbonyl-3 hydroxy-4 tétradécyl-5 isoxazole.

C'est un solide blanc de point de fusion : 92°C.

Ananlyse élémentaire:

| | C | H | N |
|---|---|---|---|
| calc. : | = 67,22% | = 9,80% | = 4,13% |
| tr. | 67,02 | 10,29 | 4,28 |

RMN[1]H ($CDCl_3$, TMS δ en ppm)
Signaux caractéristiques:

$CH_2CH_2C(=O)$ : 1,71 ; $CH_2C(=O)$ : 2,78 (t) ; $CH_3OC(=O)$ : 4,03 ; OH = 6,33

RMN[13]C: ($CDCl_3$, TMS, δ en ppm)
Signaux caractéristiques:

$C_5$ = 136,28
$C_4$ = 144,92
$C_3$ = 157,97
$C_6$ = 163,09
$C_7$ = 52,82.

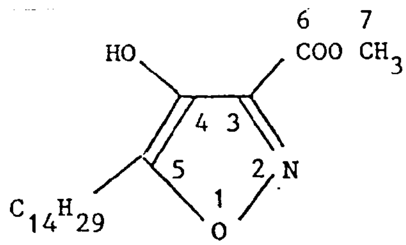

Spectre de masse:

Pic de masse par ionisation électronique : 339
Pic de masse par ionisation chimique ($CH_4$): 340
Pic de masse par ionisation chimique ($N_2O$): 339
Fragment caractéristique : $C_{14}H_{29}C=O$

Spectre UV (acétonitrile) :

λmax = 276nm ε = 2475

Du filtrat de la recristallisation on isole par HPLC l'isomère à savoir le tétradécyl-3 hydroxy-4 méthoxycarbonyl-5 isoxazole.

Il présente le même pic de masse que son isomère.

RMN[13]C :
$C_5$ = 139,50
$C_4$ = 145,74
$C_3$ = 156,80
$C_6$ = 159,89
$C_7$ = 52,38

EXEMPLE 2 : Hydrazinocarbonyl-3 hydroxy-4 tétradécyl-5 isoxazole.

Dans 50cm³ de chloroforme on dissout 13,56g (0,04mole) du composé préparé à l'exemple 1. Sous agitation et à température ambiante on ajoute 2,2g (0,044mole) d'hydrate d'hydrazine en solution dans 40cm³ d'isopropanol. Le produit formé précipite progressivement. Après quelques heures d'agitation on l'isole par essorage. Le solide est recristallisé dans 200cm³ de chloroforme. On isole 9,1g d'un solice blanc de point de fusion : 130°C.

Il présente un indice de basicité mesuré dans l'acide acétique par l'acide perchlorique de 2,9meq/g.

RMN[13]C : $C_5$ = 133,1
$C_4$ = 150,17
$C_3$ = 156,96
$C_6$ = 158,45

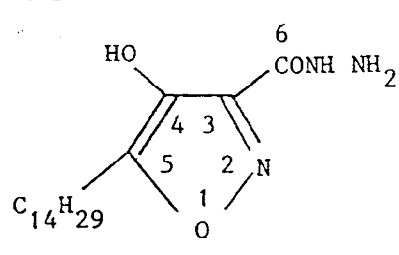

Spectre UV (acétonitrile) :

λmax = 260nm ε = 8136

EXEMPLE 3 : Chlorhydrate de (diméthylamino-2 éthylamido)-3 hydroxy-4 tétradécyl-5 isoxazole.

Dans 300cm³ d'hexane on dissout, à 60°C, 30g (0,088mole) du composé préparé selon l'exemple 1. A cette température on ajoute 7,75g (0,088mole) de diméthylamino-2 éthylamine fraîchement distillée. On porte le milieu réactionnel pendant 8 heures à reflux en éliminant le méthanol formé à l'aide d'un Dean-Stark. Après refroidissement dans la glace le milieu réactionnel conduit à 35g d'un solide que l'on recristallise dans l'hexane.

On solubilise 15g du solide recristallisé dans 100cc d'éther anhydre auquel on ajoute 5cc d'éther saturé de gaz chlorhydrique. On obtient 13g de chlorhydrate se présentant sous forme d'un solide blanc de point de fusion : 77°C.

RMN $^{13}C$ : $C_5$ = 134,89 $C_7$ = 34,46
$C_4$ = 147,51 $C_8$ = 56,96
$C_3$ = 158,26 $C_{9,10}$ = 43,72
$C_6$ = 162,06

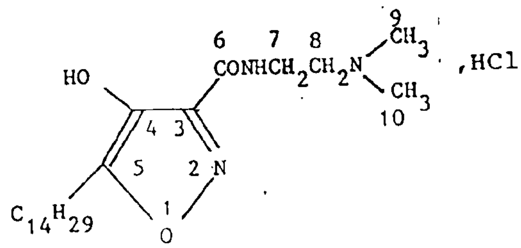

Spectre UV (acétonitrile):

λmax = 272nm ε = 2571

EXEMPLE 4 : Méthylsulfate de (triméthylammonio-2 éthylamido)-3 hydroxy-4 tétradécyl-5 isoxazole.

Dans 90cm³ d'acétone anhydre on solubilise 10g (0,025mole) du composé préparé selon l'exemple 3 isolé avant l'acidification à l'acide chlorhydrique. A cette solution on ajoute en 30 minutes et à 40°C 3,2g (0,025mol) de sulfate de diméthyle. On maintient l'agitation à cette température pendant 1 heure puis on abandonne toute une nuit au repos. Après refroidissement dans la glace on isole, par filtration, le précipité formé. On obtient 11,1g d'un solide blanc.

RMN $^{13}C$ : $C_5$ = 135,07 $C_7$ = 33,85
$C_4$ = 147,18 $C_8$ = 64,58
$C_3$ = 158,03 $C_{9,10,11}$ = 53,71
$C_6$ = 162,07 $C_{12}$ = 54,58

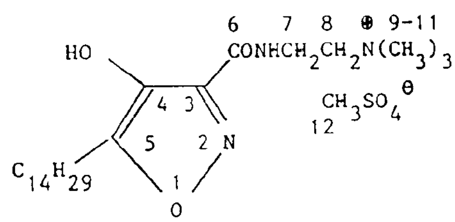

Spectre UV (acétonitrile) :

λmax = 280 nm ε = 3506

EXEMPLE 5 : n-butylamido-3 hydroxy-4 tétradécyl-5 isoxazole.

Dans 100cm³ d'hexane on dissout à 60°C 10g (0,029mole) de composé préparé à l'exemple 1. A cette température on ajoute 5g (0,068mole) de n-butylamine puis on chauffe le milieu réactionnel, 5 heures, au reflux du solvant. On élimine ensuite la butylamine en excès ainsi que le solvant par évaporation sous vide.

Le résidu de la distillation est soumi à une HPLC (dichlorométhane: hexane) sur silice. On isole 8,8g d'un solide blanc de point de fusion: 64°C.

RMN $^{13}C$ : $C_5$ = 136,22 $C_7$ = 38,85
$C_4$ = 145,94 $C_8$ = 31,40
$C_3$ = 156,79 $C_9$ = 20,01
$C_6$ = 161,90 $C_{10}$ = 13,65

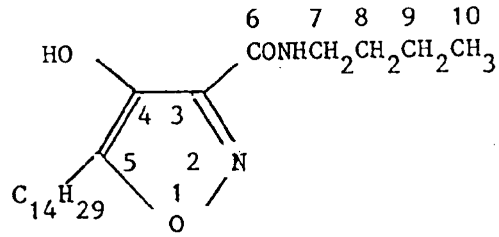

Spectre UV (acétonitrile) :

λmax = 280 nm ε = 4064

EXEMPLE 6 : Ethoxycarbonyl-3 hydroxy-4 tétradécyl-5 isoxazole

Dans 1000ml d'éthanol absolu on dissout 20g (0,059 mole) du mélange d'isomères obtenu suivant l'exemple 1. Cette solution, après addition de 20ml d'$H_2SO_4$(8N) est portée à reflux pendant une dizaine d'heures. On ajoute alors 250ml d'eau et 100ml d'$H_2SO_4$(8N) et on évapore sous vide. On extrait la solution à deux reprises avec 250ml de dichlorométhane. Après séchage sur sulfate de sodium, le solvant est éliminé sous pression réduite. Le résidu est soumis à une HPLC sur silice avec le dichlorométhane comme éluant. On isole 12,2g d'un solide blanc de point de fusion : 54°C.

RMN $^{13}C$: ($CDCL_3$ , TMS , δ en ppm).
$C_5$ = 136,35
$C_4$ = 145,03
$C_3$ = 157,87
$C_6$ = 162,82
$C_7$ = 62,54
$C_8$ = 14,13

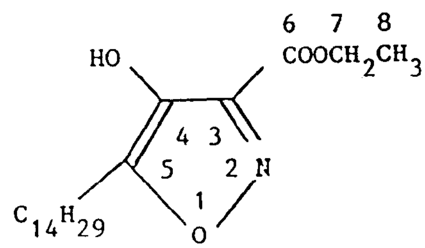

Spectre UV (acétonitrile):

λmax = 280nm ε = 2475

EXEMPLE 7 : Hexadécyloxycarbonyl-3 hydroxy-4 tétradécyl-5 isoxazole

Dans 15ml de toluène anhydre on dissout 1,7g (0,005mole) du mélange d'isomères obtenus suivant l'exemple 1. A cette solution on ajoute 3,6g (0,015mole) d'hexadécanol et 0,076g d'acide paratoluène sulfonique. On chauffe la solution à reflux du solvant pendant 4 heures. Après refroidissement le mélange abandonne un solide qui est recristallisé dans l'acétate d'éthyle. On isole 2g d'un solide blanc de point de fusion: 70°C.
RMN $^{13}C$: ($CDCL_3$ , TMS , δ en ppm).

$C_5$ = 136,36
$C_4$ = 145,01
$C_3$ = 157,79
$C_6$ = 162,92
$C_7$ = 66,59

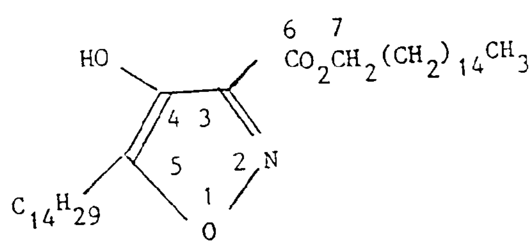

Spectre UV (acétonitrile):

λmax = 280mm ε = 3206

EXEMPLES DE COMPOSITIONS

EXEMPLE A

EXEMPLES DE COMPOSITIONS

EXEMPLE A

Crème solaire sous forme d'une émulsion huile-dans-l'eau.

- Ethoxycarbonyl -3 hydroxy-4 tétradécyl-5 isoxazole .................................. 3g
- Mélange d'alcools cétylstéarylique et cétylstéarylique à 33 moles d'oxyde d'éthylène vendu sous la dénomination de SINNOWAX AO par la Société HENKEL...................... 7,5g
- Mélange de mono et de distéarate de glycérol vendu sous la dénomination de GELEOL COPEAUX par la Société GATTEFOSSE................. 2g
- Alcool cétylique.......................... 1,8g
- Benzoate d'alcools en $C_{12}$-$C_{15}$ vendu sous la dénomination de FINSOLV TN par la Société FINETEX.......................... 10g
- Myristate d'isopropyle.................... 2,2g
- Glycérine................................. 7g
- Propylèneglycol........................... 3g
- Conservateurs............................. 0,4g
- Eau déminéralisée q.s.p................... 100g

EXEMPLE B

Huile solaire

| | |
|---|---|
| - Ethoxycarbonyl-3 hydroxy-4 tétradécyl-5 isoxazole.................................... | 2g |
| - Benzoate d'alcools en $C_{12}$-$C_{15}$ vendu sous la dénomination de FINSOLV TN par la Société FINETEX.................... | 30g |
| - Cocoate d'éthyl-2 hexyle.................. | 12g |
| - Huile de colza............................ | 10g |
| - Cyclotétradiméthylsiloxane vendu sous la dénomination de ABIL K4 par la Société GOLDSCHMIDT....................... | 14g |
| - Antioxydant.............................. | 0,04g |
| - Huile de tournesol q.s.p.................. | 100g |

**Revendications**

1. Dérivés d'hydroxy-4 isoxazoles, caractérisés par le fait qu'ils correspondent à la formule générale suivante:

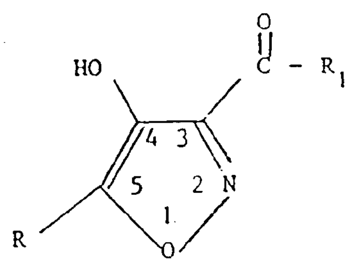

(I)

dans laquelle:

R représente un radical alkyle ou hydroxyalkyle, linéaire ou ramifié, ayant de 1 à 23 atomes de carbone, un radical alkényle, linéaire ou ramifié, ayant de 2 à 23 atomes de carbone, un radical phényle ou un radical benzyle, et

$R_1$ représente $OR_2$ , $-NHR_3$ ou $-NH-NHR_4$ ,

$R_2$ représentant un atome d'hydrogène ou un radical alkyle ayant de 1 à 24 atomes de carbone,

$R_3$ représentant un atome d'hydrogène, un radical alkyle ayant de 1 à 20 atomes de carbone ou un radical

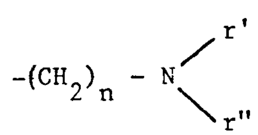

n étant 2 ou 3 et r' et r", identiques ou différents, représentant un atome d'hydrogène, un radical alkyle inférieur ou pris ensemble forment une morpholine, pipéridine ou pipérazine,

$R_4$ représentant un atome d'hydrogène ou un radical benzyle,

et les sels des composés de formule (I).

2. Composés selon la revendication 1, caractérisés par le fait que le radical alkyle ayant de 1 à 23 atomes de carbone est le radical éthyle, propyle, hexyle, éthyl-2 hexyle, octyle, nonyle, dodécyle, tétradécyle ou octadécyle.

3. Composés selon la revendication 1 ou 2, caractérisés par le fait que le radical alkyle a de préférence de 12 à 18 atomes de carbone et est pris dans le groupe constitué par le radical dodécyle, tétradécyle et octadécyle.

4. Composés selon l'une quelconque des revendications 1 à 3, caractérisés par le fait qu'ils se présentent sous forme d'un sel d'ammonium quaternaire lorsque $R_1$ représente le radical -NH-$R_3$ , $R_3$ représentant le radical

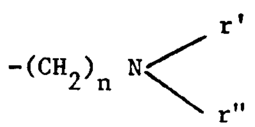

n, r' et r" étant tels que définis à la revendication 1, ces derniers ne pouvant toutefois représenter un atome d'hydrogène.

5. Composés selon l'une quelconque des revendications 1 à 4, caractérisés par le fait qu'ils sont:
   - le méthoxycarbonyl-3 hydroxy-4 tétradécyl-5 isoxazole,
   - l'hydrazinocarbonyl-3 hydroxy-4 tétradécyl-5 isoxazole,
   - le (diméthylamino-2 éthylamido)-3 hydroxy-4 tétradécyl-5 isoxazole (et son chlorhydrate),
   - le méthylsulfate de (triméthylammonio-2 éthylamido)-3 hydroxy-4 tétradécyl-5 isoxazole,
   - le n-butylamido-3 hydroxy-4 tétradécyl-5 isoxazole,
   - l'éthoxycarbonyl-3 hydroxy-4 tétradécyl-5 isoxazole, et
   - l'hexadécyloxycarbonyl-3 hydroxy-4 tétradécyl-5 isoxazole.

6. Procédé de préparation des composés de formule (I) selon l'une quelconque des revendications 1 à 5, caractérisé par le fait qu'il consiste à faire réagir du nitrite de butyle sur un β-cétoester de formule:

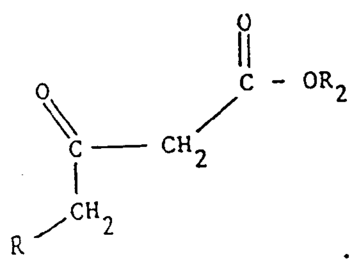

(1)

dans laquelle:

R a la même signification qu'à la revendication 1 et $R_2$ représente un radical alkyle inférieur ayant de 1 à 3 atomes de carbone, et que si désiré on transforme selon les méthodes connues l'alkoxycarbonyl-3 hydroxy-4 isoxazole obtenu en vue d'obtenir les autres significations de $R_1$.

7. Procédé selon la revendication 6, caractérisé par le fait que la réaction est réalisée dans l'éther diéthylique ou diisopropylique avec au moins 3 moles de nitrite de butyle par mole du β-cétoester en présence de gaz chlorhydrique anhydre.

8. Procédé selon l'une quelconque des revendications 6 et 7, caractérisé par le fait que la réaction est réalisée à une température inférieure à 20°C et de préférence comprise entre -10 et 10°C.

**9.** Composition cosmétique anti-solaire, caractérisée par le fait qu'elle contient dans un véhicule cosmétique acceptable au moins un composé de formule (I) ou un de ses sels selon l'une quelconque des revendications 1 à 5 ou obtenu selon l'une quelconque des revendications 6 à 8.

**10.** Composition selon la revendication 9, caractérisée par le fait que le composé de formule (I) ou un de ses sels est présent à une concentration comprise entre 0,1 et 80% en poids par rapport au poids total de la composition.

## Patentansprüche

**1.** 4-Hydroxy-Isoxazol-Derivate, dadurch gekennzeichnet, daß sie die folgende allgemeine Formel aufweisen:

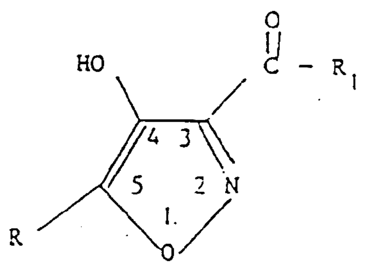

(I) ,

in der R einen linearen oder verzweigten Alkyl- oder Hydroxyalkylrest mit 1 bis 23 Kohlenstoffatomen, einen linearen oder verzweigten Alkenylrest mit 2 bis 23 Kohlenstoffatomen, einen Phenyl- oder einen Benzylrest bedeutet und

$R_1$ $OR_2$, $-NHR_3$ oder $-NH-NHR_4$ bedeutet,

$R_2$ ein Wasserstoffatom oder einen Alkylrest mit 1 bis 24 Kohlenstoffatomen bedeutet,

$R_3$ ein Wasserstoffatom, einen Alkylrest mit 1 bis 20 Kohlenstoffatomen oder einen Rest

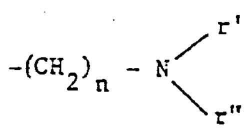

bedeutet,
worin n 2 oder 3 ist und r' und r'', die gleich oder verschieden sind, ein Wasserstoffatom, einen Niedrigalkylrest bedeuten oder zusammengenommen einen Morpholin-, Piperidin- oder Piperazinrest bilden,

$R_4$ ein Wasserstoffatom oder einen Benzylrest bedeutet,

und die Salze der Verbindungen der Formel (I).

**2.** Verbindungen nach Anspruch 1, dadurch gekennzeichnet, daß der Alkylrest mit 1 bis 23 Kohlenstoffatomen ein Ethyl-, Propyl-, Hexyl-, 2-Ethyl-Hexyl-, Octyl-, Nonyl-, Dodecyl-, Tetradecyl- oder Octadecylrest ist.

**3.** Verbindungen nach Anspruch 1 oder 2, dadurch gekennzeichnet, daß der Alkylrest vorzugsweise 12 bis 18 Kohlenstoffatome aufweist und aus der Gruppe bestehend aus Dodecyl-, Tetradecyl- und Octadecylrest ausgewählt ist.

**4.** Verbindungen nach einem der Ansprüche 1 bis 3, dadurch gekennzeichnet, daß sie in Form eines quaternären Ammoniumsalzes vorliegen, wenn $R_1$ den Rest $NH-R_3$, $R_3$ den Rest

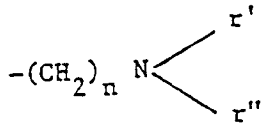

bedeutet, worin n, r' und r" die in Anspruch 1 angegebene Bedeutung aufweisen, wobei die letzteren jedoch nicht ein Wasserstoffatom bedeuten können.

5. Verbindungen nach einem der Ansprüche 1 bis 4, dadurch gekennzeichnet, daß sie die folgenden sind:
   - 3-Methoxycarbonyl-4-hydroxy-5-tetradecyl-isoxazol,
   - 3-Hydrazinocarbonyl-4-hydroxy-5-tetradecyl-isoxazol,
   - 3-(2-Dimethylamino-ethylamido)-4-hydroxy-5-tetradecyl-isoxazol (und das Chlorhydrat),
   - 3-(2-Trimethylammonio-ethylamido)-4-hydroxy-5-tetradecyl-isoxazol,
   - 3-n-Butylamido-4-hydroxy-5-tetradecyl-isoxazol-methylsulfat,
   - 3-Ethoxycarbonyl-4-hydroxy-5-tetradecyl-isoxazol, und
   - 3-Hexadecyloxycarbonyl-4-hydroxy-5-tetradecyl-isoxazol.

6. Verfahren zur Herstellung der Verbindungen der Formel (I) nach einem der Ansprüche 1 bis 5, dadurch gekennzeichnet, daß man Butylnitrit mit einem β-Ketoester der Formel:

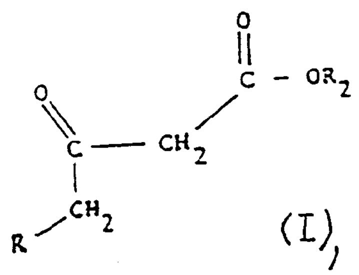

(I),

in der :

R die gleiche Bedeutung wie in Anspruch 1 aufweist und $R_2$ einen Niedrigalkylrest mit 1 bis 3 Kohlenstoffatomen bedeutet, umsetzt und, wenn gewünscht, das erhaltene 3-Alkoxycarbonyl-4-hydroxy-isoxazol nach bekannten Verfahren umwandelt, um die anderen Bedeutungen von $R_1$ zu erhalten.

7. Verfahren nach Anspruch 6, dadurch gekennzeichnet, daß man die Reaktion in Diethyl- oder Diisopropyläther mit mindestens 3 Mol Butylnitrit pro Mol β-Ketoester in Gegenwart von wasserfreiem Chlorwasserstoffgas durchführt.

8. Verfahren nach einem der Ansprüche 6 und 7, dadurch gekennzeichnet, daß man die Reaktion bei einer Temperatur unter 20°C und vorzugsweise zwischen -10°C und 10°C durchführt.

9. Kosmetische Sonnenschutzzusammensetzung, dadurch gekennzeichnet, daß sie in einem kosmetisch annehmbaren Träger mindestens eine Verbindung der Formel (I) oder eines der Salze nach einem der Ansprüche 1 bis 5 oder erhalten nach einem der Ansprüche 6 bis 8 enthält.

10. Zusammensetzung nach Anspruch 9, dadurch gekennzeichnet, daß die Verbindung der Formel (I) oder eines der Salze in einer Konzentration zwischen 0,1 und 80 Gew.-%, bezogen auf das Gesamtgewicht der Zusammensetzung, vorhanden ist.

## Claims

1. Derivatives of 4-hydroxyisoxazoles, characterised in that they correspond to the following general formula:

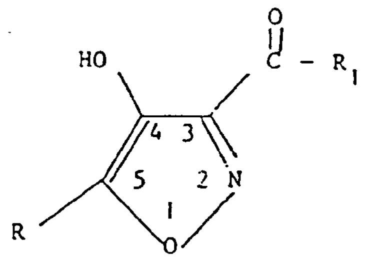

(I)

(I)

in which:
R represents a straight-chain or branched alkyl or hydroxyalkyl radical having from 1 to 23 carbon atoms, a straight-chain or branched alkenyl radical having from 2 to 23 carbon atoms, a phenyl radical or a benzyl radical, and
$R_1$ represents $OR_2$, $-NHR_3$ or $-NH-NHR_4$,
$R_2$ representing a hydrogen atom or an alkyl radical having from 1 to 24 carbon atoms,
$R_3$ representing a hydrogen atom or an alkyl radical having from 1 to 20 carbon atoms or a radical

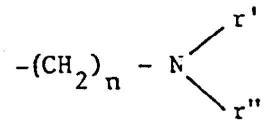

n being 2 or 3 and r′ and r″, which may be identical or different, representing a hydrogen atom or a lower alkyl radical, or taken together form a morpholine, piperidine or piperazine, and
$R_4$ representing a hydrogen atom or a benzyl radical, and the salts of the compounds of formula (I).

**2.** Compounds according to Claim 1, characterised in that the alkyl radical having from 1 to 23 carbon atoms is the ethyl, propyl, hexyl, 2-ethylhexyl, octyl, nonyl, dodecyl, tetradecyl or octadecyl radical.

**3.** Compounds according to Claim 1 or 2, characterised in that the alkyl radical preferably has from 12 to 18 carbon atoms and is taken from the group comprising the dodecyl, tetradecyl and octadecyl radical.

**4.** Compounds according to any one of Claims 1 to 3, characterised in that they are in the form of a quaternary ammonium salt when $R_1$ represents the radical $-NH-R_3$, $R_3$ representing the radical

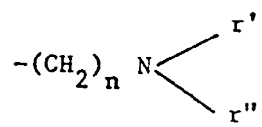

n, r′ and r″ being as defined in Claim 1, but these latter radicals not being able to represent a hydrogen atom.

**5.** Compounds according to any one of Claims 1 to 4, characterised in that they are:
- 3-methoxycarbonyl-4-hydroxy-5-tetradecylisoxazole,
- 3-hydrazinocarbonyl-4-hydroxy-5-tetradecylisoxazole,
- 3-(2-dimethylaminoethylamido)-4-hydroxy-5-tetradecylisoxazole (and its hydrochloride),
- 3-(2-trimethylammonioethylamido)-4-hydroxy-5-tetradecylisoxazole methyl sulphate
- 3-n-butylamido-4-hydroxy-5-tetradecylisoxazole,
- 3-ethoxycarbonyl-4-hydroxy-5-tetradecylisoxazole, and
- 3-hexadecyloxycarbonyl-4-hydroxy-5-tetradecylisoxazole.

**6.** Process for the preparation of compounds of formula (I) according to any one of Claims 1 to 5, characterised in that it consists in reacting butyl nitrite with a β-ketoester of formula:

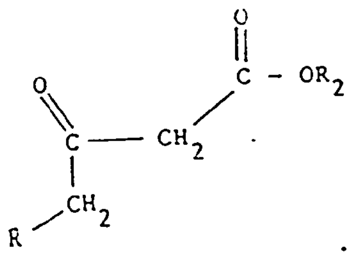

(1)

in which
R has the same meaning as in Claim 1 and $R_2$ represents a lower alkyl radical having from 1 to 3 carbon atoms, and in that, if desired, the 3-alkoxycarbonyl-4-hydroxyisoxazole obtained is converted using known methods with a view to obtaining other meanings of $R_1$.

7. Process according to Claim 6, characterised in that the reaction is carried out in diethyl ether or diisopropyl ether with at least 3 moles of butyl nitrite per mole of β-ketoester in the presence of anhydrous hydrogen chloride gas.

8. Process according to either of Claims 6 and 7, characterised in that the reaction is carried out at a temperature lower than 20°C and preferably between -10 and 10°C.

9. Sunscreen cosmetic composition, characterised in that it contains, in a cosmetically acceptable vehicle, at least one compound of formula (I), or one of its salts, according to any one of Claims 1 to 5 or obtained according to any one of Claims 6 to 8.

10. Composition according to Claim 9, characterized in that the compound of formula (I) or one of its salts is present in a concentration of between 0.1 and 80 % by weight relative to the total weight of the composition.